Europäisches Patentamt

European Patent Office

Office européen des brevets

(19)

(11) Veröffentlichungsnummer: **0 307 708**
**A1**

# (12) EUROPÄISCHE PATENTANMELDUNG

(21) Anmeldenummer: **88114079.2**

(22) Anmeldetag: **29.08.88**

(51) Int. Cl.⁴: **A61B 17/39**

(30) Priorität: **11.09.87 DE 3730604**

(43) Veröffentlichungstag der Anmeldung:
**22.03.89 Patentblatt 89/12**

(84) Benannte Vertragsstaaten:
**AT BE DE FR GB IT**

(71) Anmelder: **Siemens Aktiengesellschaft Berlin
und München
Wittelsbacherplatz 2
D-8000 München 2(DE)**

(72) Erfinder: **Hagen, Uwe, Dipl.-Ing.(FH)
Auf der Hut 25
D-8550 Forchheim(DE)**
Erfinder: **Redler, Udo, Dipl.-Ing.
Hans-Sachs-Strasse 25
D-8521 Effeltrich(DE)**

(54) **Neutrale Elektrode für ein Hochfrequenz-Chirurgiegerät.**

(57) Die neutrale Elektrode (2) für ein HF-Chirurgie-gerät umfaßt drei Teilelektroden (4a, 6a, 8a), die in einer vorgegebenen Richtung (y-Achse) auf einem isolierenden Träger (10) angeordnet sind. Der Träger (10) besitzt an einer Seite (10a), die parallel zur vorgegebenen Richtung (y) liegt, einen elektrischen Leitungsanschluß (20). Dieser enthält elektrische Lei-tungen (24, 26, 28), die zu den Teilelektroden ge-führt sind. Nach der Erfindung nimmt die Größe der Flächen der Teilelektroden (4a, 6a, 8a) in der vorge-gebenen Richtung (y) ab. Diese Elektrode wird am Patienten (1) so appliziert, daß die vorgegebene Richtung (y) auf das Operationsfeld (30) weist. Dabei werden die Teilelektroden (4a, 6a, 8a) mit im we-sentlichen gleichen HF-Anteilen beaufschlagt, so daß eine sichere Überwachung des Anliegens möglich ist.

FIG 1

EP 0 307 708 A1

## Neutrale Elektrode für ein Hochfrequenz-Chirurgiegerät

Die Erfindung bezieht sich auf eine neutrale Elektrode für ein HF-Chirurgiegerät.

Eine neutrale Elektrode der eingangs genannten Art, die mit zwei flächenhaften Teilelektroden arbeitet, ist beispielsweise aus dem deutschen Gebrauchsmuster 82 05 363 bekannt. Bei dieser neutralen Elektrode wird mittels einer Kontrollschaltung eine Überwachung der elektrisch leitenden Verbindung mit dem Patienten durch einen niederfrequenten Kontrollstrom durchgeführt. Dieser Kontrollstrom führt von der Kontaktfläche der einen Teilelektrode durch die Haut des Patienten zu der Kontaktfläche der anderen Teilelektrode.

Eine ebenfalls zweiteilige neutrale Elektrode ist aus der DE-A-28 49 422 bekannt.

Mehrteilige Elektroden erlauben es somit, mit großer Sicherheit festzustellen, ob die betreffende neutrale Elektrode des HF-Chirurgiegerätes mit ausreichend großer Fläche am Patienten anliegt oder nicht. Eine Überwachungs- oder Sicherheitsschaltung, mit der sich das Anliegen einer dreiteiligen neutralen Elektrode auf dem Gebiet der HF-Chirurgie überwachen läßt, ist beispielsweise aus FIG 4 der DE-A-35 44 443 bekannt.

Ein Problem bei der Konstruktion einer mehrteiligen Elektrode besteht darin, eine Konfiguration zu finden, die eine mögliche Unsymmetrie der Verteilung der von der aktiven Elektrode ausgehenden Hochfrequenz auf die Teilelektroden der neutralen Elektrode weitgehend vermeidet.

Bei Verwendung einer zweiteiligen Elektrode können Fehlmessungen bei der Überwachung auftreten. Dies kann insbesondere dann von Bedeutung sein, wenn die neutrale Elektrode an einer Extremität des Patienten, wie z.B. am Arm oder am Bein des Patienten, befestigt wird. Insbesondere kann es dabei zu einer Unsymmetrie bei der Verteilung der von der aktiven Elektrode ausgehenden Hochfrequenz auf die Teilelektroden der neutralen Elektrode kommen, was zu vermeiden ist.

Aufgabe der Erfindung ist es somit, eine Elektrode der eingangs genannten Art so auszubilden, daß eine gleichmäßige Verteilung der Hochfrequenz-Energie auf ihre Teilelektroden sichergestellt ist, insbesondere auch dann, wenn die Elektrode an einer Extremität des Patienten appliziert wird.

Diese Aufgabe wird bei der eingangs genannten Elektrode erfindungsgemäß durch die im Patentanspruch 1 gekennzeichneten Merkmale gelöst. Eine solche Elektrode läßt sich am Patienten so anbringen, daß jede der Teilelektroden von der vom Operationsfeld herrührenden Hochfrequenz praktisch einen gleichen Anteil erhält.

Nach der Erfindung ist vorgesehen, daß die Teilelektroden in der vorgegebenen Richtung eine kleiner werdende Flächengröße besitzen. Diese Elektrode wird am Patienten so appliziert, daß die vorgegebene Richtung im wesentlichen auf das Operationsfeld zeigt. In diesem Falle werden die unterschiedlich großen Teilflächen bei geeigneter Dimensionierung mit etwa gleichem HF-Anteil versorgt.

Weitere vorteilhafte Ausgestaltungen der Erfindung sind in den Unteransprüchen gekennzeichnet.

Ein Ausführungsbeispiel der Erfindung wird im folgenden anhand der Zeichnung näher erläutert. Es zeigen:

FIG 1 eine Aufsicht auf einen Patienten, an dessen Oberschenkel eine erfindungsgemäße dreiteilige neutrale Elektrode befestigt ist, und

FIG 2 eine neutrale Elektrode mit drei Teilelektroden unterschiedlicher Größe gemäß Fig. 1.

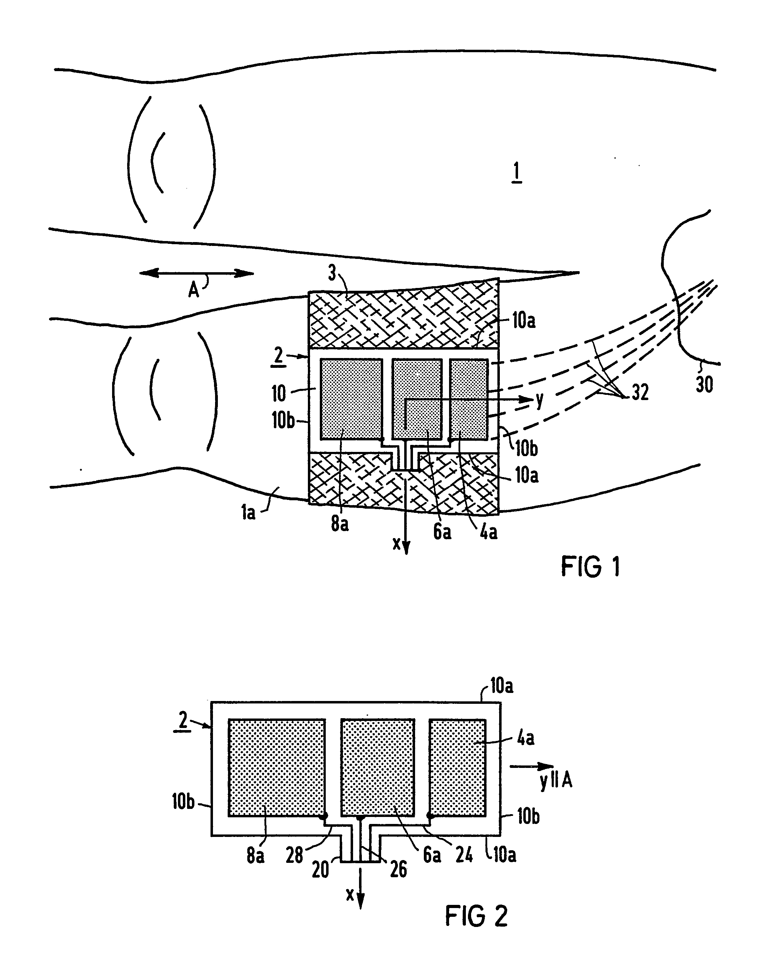

In den Figuren 1 und 2 bezieht sich das x-, y-Koordinatensystem jeweils auf die Körperachse A eines Patienten 1; die y-Achse liegt parallel zur Körperachse A.

Nach FIG 1 ist am Oberschenkel 1a eines Patienten 1 eine neutrale Elektrode 2 mittels eines Bandes 3 oder in Selbstklebetechnik befestigt. Die neutrale Elektrode 2 ist hier von der Unterseite gezeigt, um den Aufbau deutlich werden zu lassen. Die neutrale Elektrode 2 umfaßt drei flächenhafte Teilelektroden 4a, 6a und 8a, von denen je zwei durch einen Isolierstreifen oder Streifen geringerer elektrischer Leitfähigkeit voneinander getrennt sind. Die drei Teilelektroden 4a, 6a, 8a sind in einer vorgegebenen Richtung y nebeneinander angeordnet. Sie bestehen jeweils aus einer Metallfolie oder aus einem Metallnetz und sind auf einem biegsamen Träger 10 befestigt. Dieser Träger 10, der allein oder mit der gesamten Elektrode selbstklebend ausgeführt sein kann, ist im wesentlichen rechteckig ausgebildet, ist in der vorgegebenen y-Richtung länger als in der dazu senkrechten x-Richtung und steht am Rande über die drei Teilelektroden 4a, 6a und 8a über. Seine beiden Seiten 10b sind kürzer als die beiden Seiten 10a, die parallel zur y-Richtung liegen. Er besteht z.B. aus einem Gummi.

Der Träger 10 besitzt an seiner einen Seite 10a, die im wesentlichen parallel zur vorgegebenen y-Richtung liegt, einen elektrischen Leitungsanschluß 20. Dieser Leitungsanschluß 20 enthält in paralleler Führung drei Verbindungsleitungen 24, 26 und 28 für die Teilelektroden 4a, 6a bzw. 8a. Wie ersichtlich, sind die Zuleitungen 24, 28 für die beiden Teilelektroden 4a bzw. 8a jeweils auf dem Träger 10 benachbart zur langen Seite 10a geführt. Die Anschlußpunkte aller Leitungen 24, 26, 28 liegen auf einer Linie parallel zur y-Achse. Die beiden Verbindungsleitungen 24, 28 sind gleich lang, was die Herstellung der Elektrode 2 vereinfacht und die Vorratskosten gering hält. Der elektrische Leitungsanschluß 20 kann dabei jeweils als Abgriff ausgebildet sein, und zwar dergestalt, daß an ihn eine mehrpolige Klemme, beispielsweise nach Art einer Krokodilsklemme, angeschlossen wird.

In FIG 1 ist das Operationsgebiet im Patienten 1 mit 30 gekennzeichnet. Hier setzt die (nicht gezeigte) aktive Elektrode beim Schneiden oder Koagulieren an. Infolge der Anordnung der Elektrode 2 so, daß die vorgegebene Richtung x quer zum Operationsgebiet 30 und auch quer zur Körperachse A liegt, ist gewährleistet, daß sich die vom Operationsgebiet 30 ausgehende HF-Leistung wenigstens weitgehend gleichmäßig auf die Teilelektroden 4a, 6a, 8a verteilt. Die auf die neutrale Elektrode 2 übertragene HF-Leistung ist durch gestrichelte Linien 32 angedeutet.

In FIG 2 ist die gleiche Ausführungsform wie in Figur 1 dargestellt, bei der die vorgegebene Richtung die y-Achse ist, die auch hier wieder parallel zur Körperachse A des Patienten 1 liegt. Diese neutrale Elektrode 2 wird am Patienten 1 so appliziert, daß ihre y-Achse parallel zur Körperachse A liegt. Die drei rechteckig ausgebildeten Teilelektroden 4a, 6a, 8a sind entlang der y-Achse aufgereiht. Bemerkenswert ist, daß hier nicht eine gleich große Flächengröße der gleich breiten Teilelektroden 4a, 6a, 8a gewählt ist. Vielmehr sind die Teilelektroden 4a, 6a, 8a in der vorgegebenen y-Richtung jeweils mit einer kleiner werdenden Flächengröße ausgestattet. Dadurch ist bei der gezeigten Orientierung der neutralen Elektrode 2 zu dem auch hier rechts liegenden (nicht gezeigten) Operationsgebiet 30 gewährleistet, daß die einzelnen Teilelektroden 4a, 6a, 8a im wesentlichen mit demselben Hochfrequenzanteil belegt werden, so daß ein sicherer Nachweis mittels einer Überwachungsschaltung betreffend das Anliegen am Patienten 1 gewährleistet ist.

## Ansprüche

1. Neutrale Elektrode für ein Hochfrequenz-Chirurgiegerät, **dadurch gekennzeichnet**, daß die neutrale Elektrode (2) mindestens aus drei Teilelektroden (4a, 6a, 8a) besteht, daß die Teilelektroden in einer vorgegebenen Richtung (y; x) nebeneinander auf einem Träger (10) angeordnet sind, daß die Teilelektroden (4a, 6a, 8a) in der vorgegebenen Richtung (y) eine kleiner werdende Flächengröße besitzen, daß der Träger (10) an einer Seite (10a), die im wesentlichen parallel zur vorgegebenen Richtung (y) liegt, einen elektrischen Leitungsanschluß (20) besitzt, zu dem von den einzelnen Teilelektroden (4a, 6a, 8a) elektrische Verbindungsleitungen (24, 26, 28) geführt sind.

2. Elektrode nach Anspruch 1, **dadurch gekennzeichnet**, daß sie in der vorgegebenen Richtung (y) länger ist als senkrecht dazu.

3. Elektrode nach Anspruch 1, **dadurch gekennzeichnet**, daß die Teilelektroden (4a, 6a, 8a) eine im wesentlichen rechteckige Gesamtauflagefläche mit zwei parallelen kurzen und zwei parallelen langen Seiten (10b, 10a) bilden und daß der elektrische Leitungsanschluß (20) an einer der beiden langen Seiten (10a) vorgesehen ist.

4. Elektrode nach Anspruch 1, **dadurch gekennzeichnet**, daß der elektrische Leitungsanschluß (20) zum Abgriff mittels einer mehrpoligen Klemme ausgebildet ist.

FIG 1

FIG 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CA-A-1 219 642  (M. FRIZE et al.) <br> --- | 1 | A 61 B  17/39 |
| A | GB-A-1 333 573  (H. MILHENCH) <br> ----- | 1 | |
| | | | **RECHERCHIERTE SACHGEBIETE (Int. Cl.4)** |
| | | | A 61 B |

Der vorliegende Recherchenbericht wurde für alle Patentansprüche erstellt

| Recherchenort | Abschlußdatum der Recherche | Prüfer |
|---|---|---|
| DEN HAAG | 26-10-1988 | VEREECKE A. |